# EUROPÄISCHE PATENTSCHRIFT

(11) **EP 0 572 812 B1**
(45) Veröffentlichungstag und Bekanntmachung des Hinweises auf die Patenterteilung: **13.03.1996**
(21) Anmeldenummer: 93107248.2
(22) Anmeldetag: 05.05.1993
(51) Int. Cl.: C07C 29/141, C07C 31/20

(54) **Verfahren zur Herstellung von 1,3-Propandiol durch Hydrierung von Hydroxypropionaldehyd**
Method of producing 1,3-propanediol, by hydrogenation of hydroxypropionaldehyde
Procédé de préparation de 1,3-propanediol par hydrogénation de la hydroxypropionaldéhyde

(30) Priorität: 03.06.1992 DE 4218282
(43) Veröffentlichungstag der Anmeldung: 08.12.1993
(73) Patentinhaber: Degussa Aktiengesellschaft, D-60311 Frankfurt (DE)
(72) Erfinder: Haas, Thomas, Dr., W-6000 Frankfurt/Main 1 (DE); Wiegand, Norbert, Dr., W-7888 Rheinfelden (DE); Arntz, Dietrich, Dr., W-6370 Oberursel (DE)

(56) Entgegenhaltungen:
- EP-A- 0 412 337
- EP-A- 0 535 565
- FR-A- 1 335 323
- CHEMICAL ABSTRACTS, vol. 87, no. 1, 1977, Columbus, Ohio, US; abstract no. 5377, Seite 5380 ;Spalte 2 ; & JP-A-76 138 607

## Beschreibung

Die vorliegende Erfindung betrifft ein Verfahren zur Herstellung von 1,3-Propandiol mit einem Restcarbonylgehalt unter 500 ppm, berechnet als Propionaldehyd, durch katalytische Hydrierung von 3-Hydroxypropionaldehyd in wäßriger Lösung.

1,3-Propandiol besitzt als Monomerbaustein für Polyester und Polyurethane sowie als Ausgangsstoff für die Synthese zyklischer Verbindungen vielseitige Anwendungsmöglichkeiten.

Zur Herstellung von 1,3-Propandiol sind verschiedene Verfahren bekannt, die entweder von einem Molekülaufbau aus einem C₂- und C₁-Baustein oder vorzugsweise direkt von einem C₃-Baustein, wie insbesondere Acrolein, ausgehen. Bei Verwendung von Acrolein, wie beispielsweise im Verfahren gemäß der DE-OS 39 26 136 oder der DE-Patentanmeldungen P 40 38 192.7, P 41 38 981.6 und P 41 38 982.4, wird dieses in Gegenwart eines sauren Hydratisierungskatalysators in wäßriger Phase zu 3-Hydroxypropionaldehyd (HPA) hydratisiert.

Das bei der Hydratisierung von Acrolein gebildete wäßrige Reaktionsgemisch, das außer Wasser und 3-Hydroxypropionaldehyd noch nicht umgesetztes Acrolein sowie Nebenprodukte aus der Hydratisierung, wie 4-Oxaheptandial, und gegebenenfalls gelöste Hydratisierungskatalysatoren enthält, wird nach Abtrennung nicht umgesetzten Acroleins und gegebenenfalls eines Teils des Wassers in Gegenwazt von Hydrierkatalysatoren hydriert. Aus dem so erhaltenen Reaktionsgemisch wird 1,3-Propandiol mittels an sich bekannter destillativer und/oder extraktiver Methoden gewonnen.

Gemäß der US-PS 2,434,110 sind für die Hydrierung von 3-Hydroxypropionaldehyd zu 1,3-Propandiol solche Katalysatoren geeignet, welche ein oder mehrere hydrierwirksame Metalle, wie Fe, Co, Ni, Cu, Ag, Mo, W, V, Cr, Rh, Pd, Os, Ir, Ru, Pt, enthalten. Wie in der PE-OS 39 26 136 beschrieben, kann der Katalysator sowohl in suspendierter Form per se oder trägergebunden vorliegen oder Bestandteil von Festbettkatalysatoren sein; auch homogene Katalysatoren können herangezogen werden.

Bevorzugte Hydrierkatalysatoren, mit welchen ein im wesentlichen quantitativer Umsatz des 3-Hydroxypropionaldehyds zu 1,3-Propandiol möglich ist, sind a) Raney-Nickel, das mit weiteren katalytisch wirksamen Metallen dotiert sein kann, b) platinbeschichtete Trägerkatalysatoren auf der Basis von Metalloxiden oder Aktivkohle, wie insbesondere Trägerkatalysatoren auf der Basis von Titandioxid mit einer Menge Pt, relativ zum Träger, von 0,1 bis 5,0 Gew.-% in fein verteilter Form gemäß der DE-Patentanmeldung P 41 32 663.6, oder c) nickelbeschichtete oxidische oder silikatische Trägerkatalysatoren, wie insbesondere solche auf der Basis von Ni/Al₂O₃ /SiO₂. Eine hohe Raum-Zeit-Ausbeute wird bei der Hydrierung erzielt, wenn das zu hydrierende wäßrige Reaktionsgemisch mit einem HPA-Gehalt von 2 bis 80 Gew.-%, vorzugsweise 5 bis 50 Gew.-% und insbesondere 5 bis 20 Gew.-%, einem pH-Wert im Bereich von 2,5 bis 6,5 aufweist, die Hydriertemperatur im Bereich zwischen 30 und 180 °C liegt und bei Wasserstoffdruken von 5 bis 300 bar hydriert wird.

Bei der großtechnischen Herstellung von 1,3-Propandiol durch Hydrierung von 3-Hydroxypropionaldehyd in wäßriger Phase wurde festgestellt, daß selbst unter den vorgenannten optimalen Reaktionsbedingungen bei einem HPA-Umsatz von im wesentlichen 100 % und einer Selektivität von gleichfalls nahe 100 % und sorgfältiger destillativer Aufarbeitung des Reaktionsgemischs und Rein-Destillation des 1,3-Propandiols dieses einen Restcarbonylgehalt, berechnet als Propionaldehyd, von einigen tausend ppm aufweist. Dieser hohe Carbonylgehalt erwies sich bei der Herstellung von Poly(1,3-propylenglykolterephthalat) und Verwendung dieses Polymeren zur Faserherstellung nach dem Schmelzspinnverfahren als sehr störend, weil es hierdurch sowohl zu Geruehsbelästigungen als auch zu Verfärbungen der Fasern kommt.

Aufgabe der Erfindung ist demnach, ein Verfahren zur Herstellung von 1,3-Propandiol mit einem verringerten Restcarbonylgehalt, vorzugsweise einem solchen unter 500 ppm, berechnet als Propionaldehyd, aufzuzeigen, das auf der katalytischen Hydrierung von 3-Hydroxypropionaldehyd in wäßriger Phase, insbesondere einem HPA enthaltenden, von nicht umgesetztem Aerolein befreiten wäßrigen Reaktiongemisch aus der Hydratisierung von Acrolein, basiert.

Gefunden wurde ein Verfahren zur Herstellung von 1,3-Propandiol mit einem Restcarbonylgehalt unter 500 ppm, berechnet als Propionaldehyd, durch katalytische Hydrierung von 3-Hydroxypropionaldehyd (HPA) in wäßriger Lösung in Gegenwart eines Festbett- oder Suspensionshydrierkatalysators bei einem Wasserstoffdruck von 5 bis 300 bar, einem pH-Wert von 2,5 bis 6,5 und einer Temperatur von 30 bis 180 °C und Aufarbeitung des Reaktionsgemischs mittels bekannter destillativer und/oder extraktiver Maßnahmen, das dadurch gekennzeichnet ist, daß man die Hydrierung bis zu einem HPA-Umsatz im Bereich von 50 bis 95 % bei 30 bis 80 °C und die weitere Hydrierung bis zu im wesentlichen 100 %-igem HPA-Umsatz bei 100 bis 180 °C durchführt.

Bei der Hydrierung von 3-Hydroxypropionaldehyd zu 1,3-Propandiol ist zu beobachten, daß die gewünschte Reaktion linear von der Reaktionszeit bzw. der Raumgeschwindigkeit (LHSV = liquid hourly space velocity) im Falle kontinuierlicher Verfahren mit einem Festbettkatalysator abhängt, jedoch kaum von der Hydriertemperatur. Demgegenüber steigt aber die Bildung von unerwünschten Nebenprodukten bei der Erhöhung der Temperatur exponentiell an. Da der Restcarbonylgehalt im Rein-1,3-Propandiol im Falle einer Hydrierung bei durchgehend niedriger Temperatur, etwa 40 bis 60 °C, bereits unbefriedigend hoch war, war es überrasehend, daß durch das erfindungsgemäße Hydrierverfahren mit mindestens einer ersten Temperaturstufe im Bereich von 30 bis 80 °C, vorzugsweise 40 bis 70 °C, und mindestens einer zweiten Temperaturstufe im Bereich von 100 bis 180 °C, vorzugsweise 110 bis 150 °C, der Restearbonylgehalt wesentlich abgesenkt werden konnte.

Im allgemeinen liegt der Restcarbonylgehalt im erfindungsgemäß hergestellten Rein-1,3-Propandiol wesentlich unter 500 ppm, meist unter 150 ppm und vorzugsweise unter 100 ppm, jeweils berechnet als Propionaldehyd. Der Carbonylgehalt läßt sich nach üblicher überführung der Carbonylverbindungen in die Dinitrophenylhydrazone entweder in bekannter Weise gravimetrisch oder im Falle geringerer Gehalte vorzugsweise mittels HPLC und UV-Detektion bestimmen. Der so bestimmte Restcarbonylgehalt erfaßt auch in acetalisierter Form vorliegende Aldehyde, wie z. B. Acetale aus 3-Hydroxypropionaldehyd und 1,3-Propandiol.

Die Hydrierung wird in an sich bekannter Weise in bekannten Reaktoren durchgeführt wobei das flüssige Reaktionsgemisch, der Katalysator und Wasserstoff in engen Kontakt miteinander gebracht werden. Im Falle der Suspensionshydrierung eignen sich insbesondere Rührreaktoren oder Strömungsreaktoren, wobei die erfindungsgemäßen Temperaturstufen im durch orientierende Vorversuche ermittelten Zeitabstand hintereinander eingestellt oder innerhalb eines Reaktorsystems räumlich hintereinander angeordnet werden. Im Falle der Suspensionshydrierung wird bei einer Temperatur im Bereich voll vorzugsweise 40 bis 60 °C bis zu einem HPA-Umsatz von 50 bis 80 % hydriert; die weitere Hydrierung bis im wesentlichen 100 % HPA-Umsatz wird vorzugsweise bei 110 bis 150 °C ausgeführt.

Für die Hydrierung von 3-Hydroxypropionaldehyd in großtechnischem Maßstab, vorzugsweise der Hydrierung eines HPA enthaltenden, von nicht umgesetztem Acrolein befreiten, wäßrigen Reaktionsgemisch aus der Hydratisierung von Acrolein, eignet sich insbesondere die Ausführungsform in einem mit einem Festbettkatalysator ausgestatteten Festbetthydrierreaktor. In einein solchen Reaktor strömt oder rieselt das flüssige Reaktionsgemisch zusammen mit dem eingespeisten Wasserstoff über den Festbettkatalysator. Als Festbett werden sowohl. Schüttungen des geformten Katalysators (z. B. Strangpreßlinge, Pellets, Kugeln) als auch auf Formkörpern mit großer Oberfläche, etwa wabenförmigen Keramikträgern, fixierte Hydrierkatalysatoren verstanden Zum Zwecke einer guten Verteilung des Wasserstoffs im Reaktionsgemisch und einer gleichförmigen Verteilung des Gas-Flüssig-Gemisches über den gesamten Querschnitt des Festbetts können das flüssige Reaktionsgemisch und der Wasserstoff gemeinsam vor dem Katalysatorbett über statische Mischer geleitet werden. Der Reaktor wird derart ausgestattet, daß in einer ersten Zone des Katalysatorbetts, die etwa 50 bis 95 %, vorzugsweise 75 bis 90 % und insbesondere 80 bis 90 % des Volumens ausmacht, eine Reaktionstemperatur im Bereich von 30 bis 80 °C, vorzugsweise 40 bis 70 °C, und innerhalb einer zweiten Zone mit dem restlichen Volumen des Katalysatorbetts eine Reaktionstemperatur im Bereich von 100 bis 180 °C, vorzugsweise 110 bis 150 °C, eingestellt und gehalten werden kann. Die genannten Temperaturzonen können jeweils eine im wesentlichen konstante Temperatur oder innerhalb des jeweiligen Bereichs ein ansteigendes Temperaturprofil aufweisen; selbstverständlich liegt zwischen der ersten und der zweiten Temperaturzone eine Aufheizzone.

Die Raumgeschwindigkeit im Katalysatorbett wird derart eingestellt, daß bei konstanter Temperatur über das gesamte Katalysatorbett, beispielsweise 60 °C, ein im wesentlichen quantitativer Umsatz an HPA erzielt wird. Durch Unterteilung des Katalysatorbetts in die erfindungsgemäßen Temperaturzonen gelingt es dann bei gleicher Raumgeschwindigkeit, den Restcarbonylgehalt im Rein-1,3-Propandiol auf Werte unter 500 ppm und meist unter 100 ppm abzusenken.

Für das erfindungsgemäße Verfahren eignen sich vorzugsweise solche Hydrierkatalysatoren, welche sich im Stand der Technik besonders bewährt haben und im einleitenden Teil dieser Anmeldung beschrieben wurden. Besonders geeignete Katalysatoren für die Suspensionshydrierung sind Raney-Ni-katalysatoren und Trägerkatalysatoren auf der Basis Pt oder Ru auf Aktivkohle, Al₂O₃, SiO₂ oder TiO₂; für die Festbetthydrierung eignen sich insbesondere die vorgenannten Trägerkatalysatoren sowie Nickel auf oxidischen oder silikatischen Trägern. Die Hydrierbedingungen bezüglich HPA-Konzentration, H₂-Druck und pH-Wert folgen gleichfalls aus dem Stand der Technik.

Das erfindungsgemäß hergestellte 1,3-Propandiol mit einem niedrigen Restcarbonylgehalt von meist unter 100 ppm, berechnet als Propionaldehyd, eignet sich sehr gut zur Herstellung von Polypropylenglykolterephthalatfasern; es kommt bei der Polykondensation und der Verspinnung weder zu einer Geruchsbelästigung noch zu Verfärbungen. Das erfindungsgemäße Verfallren zeichnet sich außer durch diesen Qualitätsvorteil des 1,3-Propandiols durch seine einfache Ausführbarkeit aus. Unerwarteterweise werden durch die erfindungsgemäße Maßnahme die Selektivität und die Ausbeute nicht gemindert.

Das Verfahren wird durch die nachfolgenden Beispiele weiter erläutert. Als beste Ausführungsform wird jene mit einem Festbettkatalysator und insbesondere einem Pt-TiO₂-Trägerkatalysator (gemäß DE-Patentanmeldung P 41 32 663.6) oder einem Ni/Al₂O₃/SiO₂-Katalysator angesehen.

### Beispiele 1 und 2 sowie Vergleichsbeispiele 1 und 2a bis 2c

Hydrierung einer wäßrigen Lösung von 3-Hydroxypropanal (HPA) in einem Rieselbettreaktor
Die wäßrige HPA-Lösung wurde hergestellt durch Hydratisierung von Acrolein, wobei eine 16,9 gew.-%ige wäßrige Acroleinlösung unter Konstanthaltung einer Reaktionstemperatur von 60 °C mit einer Raumgesehwindigkeit (LHSV) von 0,54 Std⁻¹ über ein Ionenaustauscher-Festbett (®Lewatit TP 208 / H-Form, Firma Bayer AG) geleitet wurde; die Lösung wurde bei 65 °C unter vermindertem Druck von nicht umgesetztem Acrolein befreit.

Zur kontinuierlichen Hydrierung im Rieselbett wurde eine Rieselbettanlage mit 1,3 l Reaktorvolumen eingesetzt. Die Anlage bestand aus einer Flüssigkeitsvorlage, einer Vorbeizstrecke, dem Festbettreaktor und einem Flüssigkeitsabscheider. Mittels Wärmeträger-Ölkreisläufe wurde entlang der ersten ca. 85 % des Reaktorbettes die Reaktortemperatur T_{R1} und entlang der restlichen ca. 15 % des Reaktorbetts die Reaktortemperatur T_{R2} eingestellt. Druck und Wasserstoffstrom wurden elektronisch geregelt. Die wäßrige HPA-Lösung wurde vor der Vorheizstrecke dem Wasserstofstrom min einer Pumpe zudosiert und das Gemisch am Kopf des Reaktors aufgegeben (Rieselbettfahrweise). Nach Durchlaufen des Reaktors wurde das gebildete Produkt in regelmäßigen Abständen aus dem Abscheidegefäß entnommen und der Wasserstoff mit Hilfe eines Kompressors kontinuierlich rezykliert. Das Produkt wurde mit HPLC auf nicht umgesetztes HPA untersucht und das gebildete 1,3-Propandiol mit GC bestimmt. Aus dem hydrierten Reaktionsgemisch wurde zunächst Wasser abdestilliert und dann das Roh-1,3-Propandiol fraktioniert destilliert (Siedepunkt des Rein-1,3-Propandiol 134 °C bei 50 mbar).

In Beispiel 1 und Vergleichsbeispiel 1 (=VB1) war das Reaktorbett mit einem handelsüblichen Festbett-Nickelkatalysator in Form von Strangpreßlingen (Durchmesser 1,6 mm in VB1, 0,8 mm in Beispiel 1) vom Typ Ni/Al₂O₃/SiO₂ mit einem Ni-gehalt von etwa 30 % gefüllt.

In Beispiel 2 und den Vergleichsbeispielen 2a bis 2c (=VB 2a biS VB 2c) war das Reaktorbett mit einem Trägerkatalysator in Form von Strangpreßlingen mit einem Preßlingdurchmesser von 1,6 mm und einer Länge von 10 mm auf der Basis von pyrogenem Titandioxid mit 2 Gew.-% darauf in feinverteilter Form abgeschiedenem Platin (gemäß DE-Patentanmeldung P 41 32 663.6) gefüllt.

Die Reaktionsbedingungen während der Hydrierung und die Ergebnisse folgen aus der Tabelle 1. Hierin bedeuten P_{H2} der Wasserstoffdruck in bar; LHSV die Raumgeschwindigkeit in Std⁻¹; C_{HPA/E} bzw. C_{HPA/P} die Konzentration (Gew.-%) an HPA im Edukt beziehungsweise hydrierten Reaktionsgemisch; C_{PD/P} die Konzentrationen (Gew.-%) an Propandiol im hydrierten Reaktionsgemisch; T_{R1} und T_{R2} die Temperaturen (°C) in den Reaktorzonen 1 und 2; U = Umsatz in %, S = Selektivität in %; Carb = Restcarbonylgehalt im fraktioniert destillierten 1,3-Propandiol in ppm (part per million), berechnet als Propionaldehyd.

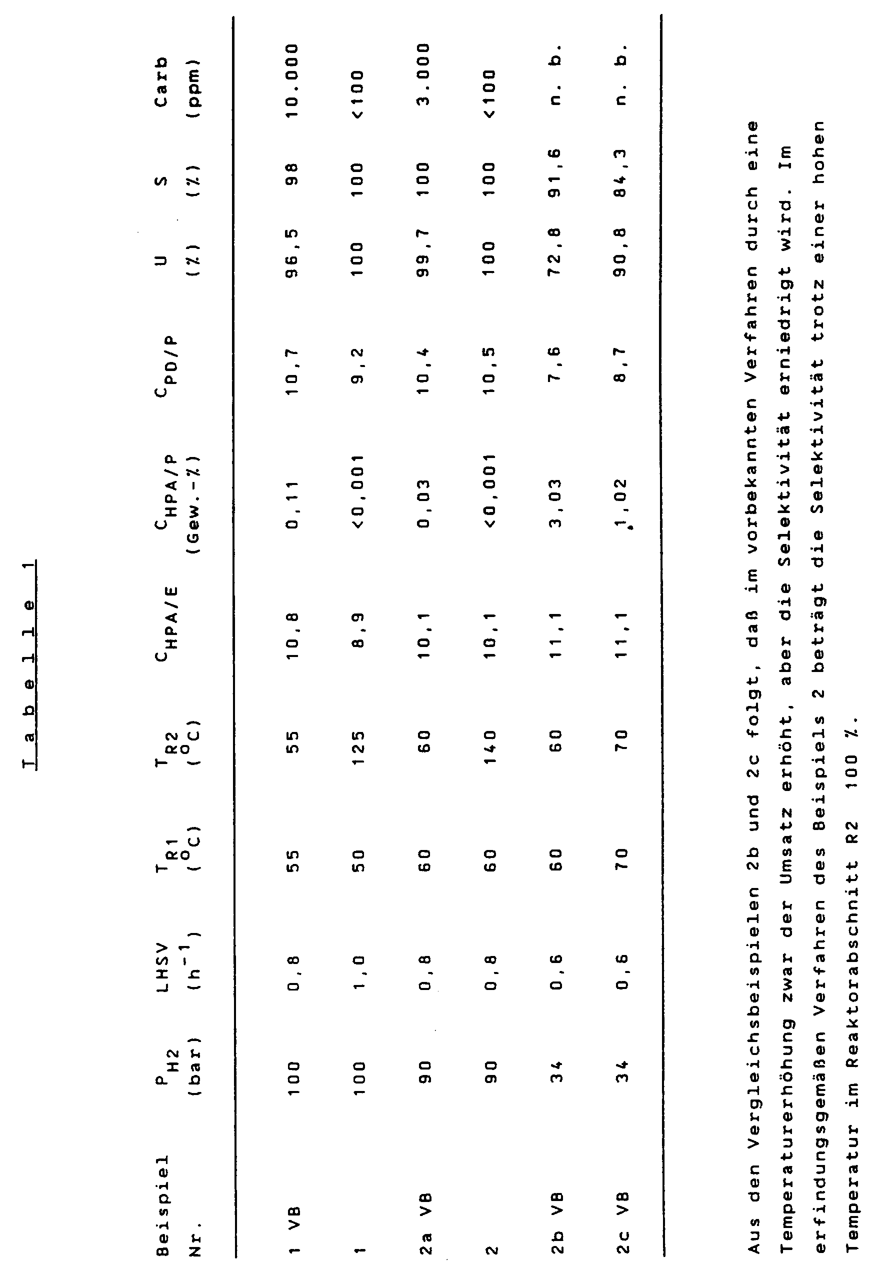

Der Vergleich des Beispiels 1VB mit 1 sowie 2VB mit 2 zeigt, daß durch das erfindungsgemäße Verfahren die Herstellung von 1,3-Propandiol mit einem Restcarbonylgehalt unter 100 ppm, berechnet als Propionaldehyd, ohne Ausbeuteverlust möglich ist.

### Beispiel 3 und Vergleichsbeispiele 3a und 3b

Hydrierung einer wäßrigen Lösung von 3-Hydroxypropionaldehyd (HPA) in einem Suspensionshydrierreaktor. Die Herstellung der wäßrigen HPA-Lösung erfolgte wie in den Beispielen 1 und 2 beziehungsweise VB1 und VB2.

500 g der HPA-Lösung werden in einem 1000 ml-Autoklaven mit Begasungsrührer unter Wasserstoffdruck bei einer bestimmten Reaktionstemperatur und einer Rührerdrehzahl von 1000 UpM in Gegenwart von 7,5 g Raney-Nickel hydriert. Die Reaktionsbedingungen und die Ergebnisse folgen aus Tabelle 2; die Angaben in der Kupfleiste der Tabelle 2 haben die gleiche Bedeutung wie in Tabelle 1, jedoch stehen T₁ und T₂ für die Temperatur (°C) während des ersten beziehungsweise zweiten Teils der Hydrierung und t₁ und t₂ für die dazugehörenden Reaktionszeiten (Min). Nach beendeter Hydrierung wurden C_{HPA/P}, C_{PD/P} bestimmt und daraus mit C_{HPA/E} der Umsatz und die Selektivität bestimmt. Vom hydrierten Reaktionsgemisch wurde Wasser abdestilliert, anschließend wurde das verbleibende Roh-1,3-Propandiol fraktioniert destilliert.

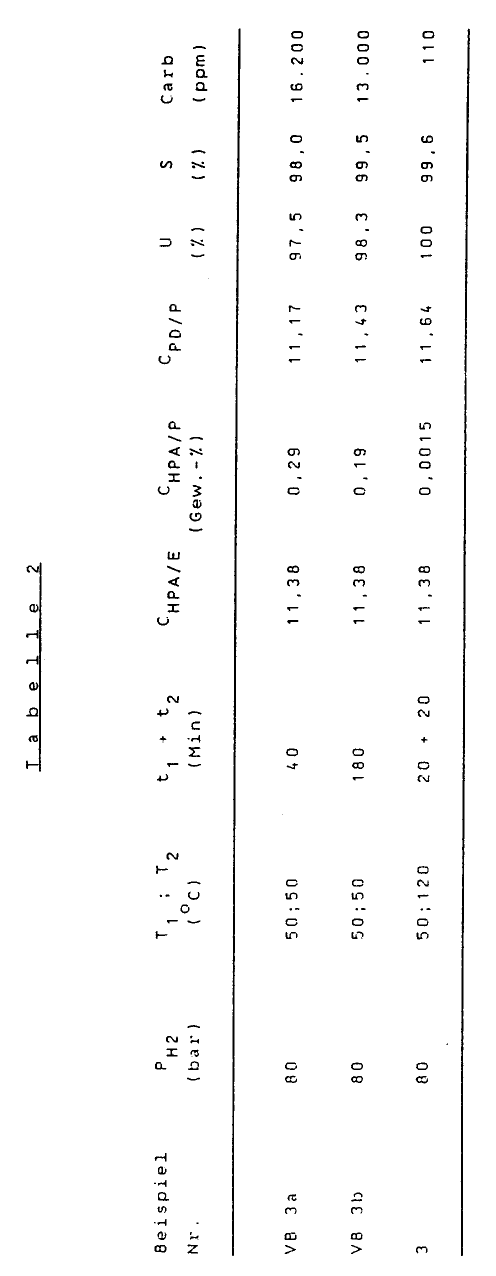

## Patentansprüche

1. Verfahren zur Herstellung von 1,3-Prupandiol mit einem Restcarbonylgehalt unter 500 ppm, berechnet als Propionaldehyd, durch katalytische Hydrierung von 3-Hydroxypropionaldehyd (HPA) in wäßriger Lösung in Gegenwart eines Festbett- oder Suspensionshydrierkatalysators bei einem Wasserstoffdruck von 5 bis 300 bar, einem pH-Wert von 2,5 bis 6,5 und einer Temperatur von 30 bis 180 °C und Aufarbeitung des Reaktionsgemischs mittels bekannter destillativer und/oder extraktiver Maßnahmen,
dadurch gekennzeichnet,
daß man die Hydrierung bis zu einem HPA-Umsatz im Bereich von 50 bis 95 % bei 30 bis 80 °C und die weitere Hydrierung bis zu im wesentlichen 100 %-igem HPA-Umsatz bei 100 bis 180 °C durchführt.

2. Verfahren nach Anspruch 1,
dadurch gekennzeichnet,
daß man die Hydrierung in einem Festbetthydrierreaktor, vorzugsweise einem Rieselbettreaktor, mit stufenweise ansteigendem Temperaturprofil innerhalb des Katalysatorbetts durchführt, wobei die Reaktionstemperatur innerhalb des ersten Teils des Katalysatorbetts, der 50 bis 95 %, vorzugsweise 75 bis 90 %, des gesamten Katalysatorbetts ausmacht, im Bereich von 30 bis 80 °C, vorzugsweise in Bereich von 40 bis 70 °C und innerhalb des restlichen Teils des Katalysatorbetts im Bereich von 100 bis 180 °C, vorzugsweise im Bereich von 110 bis 150 °C, gehalten wird.

3. Verfahren nach Anspruch 1,
dadurch gekennzeichnet,
daß man die Hydrierung in einem Suspensionshydrierreaktor, vorzugsweise einem Rührreaktor, bis zu einem HPA-Umsatz von 50 bis 80 % bei 40 bis 60 °C und die weitere Hydrierung bis zu im wesentlich 100 % HPA-Umsatz bei 110 bis 150 °C durchführt.

4. Verfahren nach einein dur Ansprüche 1 bis 3,
dadurch gekennzeichnet,
daß man einen Hydrierkatalysator aus der Reihe
a) Raney-Nickel, das auch mit anderen hydrierwirksamen Metallen dotiert sein kann, soweit es sich ausschließlich um eine Suspensionshydrierung handelt,
b) Trägerkatalysatoren auf der Basis platin- oder rutheniumbeschichteter Aktivkohle oder Metalloxide, wie insbesondere Al₂O₃, SiO₂ oder TiO₂
oder
c) nickelbeschichteter oxidischer oder silikatischer Trägerkatalysatoren, vorzugsweise Katalysatoren auf der Basis von Ni/Al₂O₃/SiO₂
verwendet.

## Claims

1. A process for the production of 1,3-propanediol having a residual carbonyl content below 500 ppm, expressed as propionaldehyde, by catalytic hydrogenation of 3-hydroxypropionaldehyde (HPA) in aqueous solution in the presence of a fixed-bed or suspension hydrogenation catalyst under a hydrogen pressure of 5 to 300 bar, at a pH value of 2.5 to 6.5 and at a temperature of 30 to 180°C and working up of the reaction mixture by known distillation-based and/or extraction-based measures, characterized in that the hydrogenation is carried out at 30 to 80°C to an HPA conversion of 50 to 95% and is then continued at 100 to 180°C to an HPA conversion of substantially 100%.

2. A process as claimed in claim 1, characterized in that the hydrogenation is carried out in the fixed-bed hydrogenation reactor, preferably a trickle-bed reactor, with a temperature profile increasing in stages in the catalyst bed, the reaction temperature in the first part of the catalyst bed, which makes up 50 to 95% and preferably 75 to 90% of the total catalyst bed, being in the range from 30 to 80°C and preferably in the range from 40 to 70°C and the reaction temperature in the remaining part of the catalyst bed being in the range from 100 to 180°C and preferably in the range from 110 to 150°C.

3. A process as claimed in claim 1, characterized in that the hydrogenation is carried out in a suspension hydrogenation reactor, preferably a stirred reactor, at 40 to 60°C to an HPA conversion of 50 to 80% and is continued at 110 to 150°C to an HPA conversion of substantially 100%.

4. A process as claimed in any of claims 1 to 3, characterized in that a hydrogenation catalyst from the series
a) Raney nickel which may also be doped with other hydrogenation-active metals providing the hydrogenation is carried out solely by suspension hydrogenation,
b) supported catalysts based on platinum- or ruthenium-coated active carbon or metal oxides, such as in particular Al₂O₃, SiO₂ or TiO₂
or
c) nickel-coated oxide- or silicate-containing supported catalysts, preferably catalysts based on Ni/Al₂O₃/SiO₂
is used.

## Revendications

1. Procédé de préparation de 1,3-propanediol ayant une teneur en carbonyle résiduelle en dessous de 500 ppm, calculé comme propionaldéhyde, par hydrogénation catalytique du 3-hydroxypropionaldéhyde (HPA) en solution aqueuse en présence d'un catalyseur d'hydrogénation en lit fixe ou en suspension à une pression d'hydrogène de 5 à 300 bars, à une valeur de pH de 2,5 à 6,5 et à une température de 30 à 180°C et traitement du mélange réactionnel à l'aide des mesures connues par distillation et/ou par extraction, caractérisé en ce que l'on effectue l'hydrogénation jusqu'à une conversion de HPA dans la zone allant de 50 à 95 % de 30 à 80°C et en ce que l'on effectue l'hydrogénation supplémentaire jusqu'à une conversion de HPA essentiellement à 100 % de 100 à 180°C.

2. Procédé selon la revendication 1, caractérisé en ce que l'on effectue l'hydrogénation dans un réacteur d'hydrogénation à lit fixe, de préférence dans un réacteur à lit de ruissellement, avec un profil de température augmentant graduellement, à l'intérieur du lit de catalyseur, dans lequel la température de réaction à l'intérieur de la première partie du lit de catalyseur, qui représente de 50 à 95 %, de préférence 75 à 90 % de la totalité du lit de catalyseur, est maintenue dans la plage allant de 30 à 80°C, de préférence dans la plage allant de 40 à 70°C et à l'intérieur de la partie restante du lit de catalyseur dans la plage de 100 à 180°C, de préférence dans la plage de 110 à 150°C.

3. Procédé selon la revendication 1, caractérisé en ce que l'on effectue l'hydrogénation dans un réacteur d'hydrogénation en suspension, de préférence un réacteur d'agitation, jusqu'à une conversion de HPA de 50 à 80 % entre 40 et 60°C et l'hydrogénation supplémentaire jusqu'à une conversion de 100 % de HPA essentiellement entre 110 et 150°C.

4. Procédé selon l'une des revendications 1 à 3, caractérisé en ce que l'on utilise un catalyseur d'hydrogénation choisi dans la série :
a) du nickel de Raney, qui peut être dopé aussi avec d'autres métaux actifs pour l'hydrogénation pour autant qu'il s'agisse exclusivement d'une hydrogénation en suspension,
b) de catalyseurs sur support à base de charbon actif ou d'oxydes métalliques recouverts de platine ou de ruthénium, comme en particulier Al₂O₃, SiO₂ ou TiO₂;
c) de catalyseurs sur support oxydiques ou silicatés, recouverts de nickel, de préférence des catalyseurs à base de Ni/Al₂O₃/SiO₂.
